# EUROPÄISCHE PATENTSCHRIFT

(11) **EP 2 564 822 B1**
(45) Veröffentlichungstag und Bekanntmachung des Hinweises auf die Patenterteilung: **16.10.2013**
(21) Anmeldenummer: 11179475.6
(22) Anmeldetag: 31.08.2011
(51) Int. Cl.: A61F 13/56, B32B 37/20

(54) **Verfahren zur Herstellung einer Materialbahn, aus der elastisch dehnbare Windelverschlusselemente ausstanzbar sind**
Method for producing a sheet of material from which elastically extendable nappy closers can be stamped
Procédé de fabrication d'une bande de matériau pouvant être découpée à partir de l'élément pour fermeture d'angle étirable de manière élastique

(43) Veröffentlichungstag der Anmeldung: 06.03.2013
(73) Patentinhaber: Mondi Consumer Packaging Technologies GmbH, 48599 Gronau (DE)
(72) Erfinder: Schönbeck, Marcus, 33775 Versmold (DE)
(74) Vertreter: Albrecht, Rainer Harald

(56) Entgegenhaltungen:
- DE-A1-102004 035 649
- US-A1- 2003 051 804

## Beschreibung

Die Erfindung betrifft ein Verfahren zur Herstellung einer Materialbahn, aus der elastisch dehnbare Windelverschlusselemente mit einem nicht elastischen und verstärkten Anschlussbereich zur Befestigung eines Verschlussteils ausstanzbar sind, wobei parallele und zueinander beabstandete Folienstreifen aus einem elastisch dehnbaren Polymer zwischen zwei Deckschichten aus Nonwoven einkaschiert werden.

Aus DE 10 2004 035 649 A1 ist ein Verfahren mit den eingangs beschriebenen Merkmalen zur Herstellung von Windelverschlusselementen bekannt. In den Bahnabschnitten zwischen den elastischen Folienstreifen werden die Nonwovenbahnen unmittelbar miteinander verklebt. Die aus der Materialbahn ausgestanzten Windelverschlusselemente weisen jeweils einen elastisch dehnbaren Abschnitt und beidseits angrenzende Anschlussbereiche aus Nonwoven auf. Die Windelverschlusselemente können als Streifen ausgebildet sein oder die Form sogenannter Windelohren aufweisen, deren Anschlussbereich an einer Windel breiter ist als der Anschlussbereich zur Befestigung des Verschlussteils. Über das Verschlussteil werden große Kräfte auf das Windelverschlusselement übertragen. Für eine gleichmäßige Krafteinleitung in das Windelverschlusselement ist ein biegesteifer Anschlussbereich hoher Zugfestigkeit vorteilhaft. Ferner ist dafür Sorge zu tragen, dass die aus Nonwoven bestehenden Anschlussbereiche nicht ausfransen oder sich plastisch dehnen, wenn die Windelverschlusselemente im Gebrauch bis zur Dehngrenze des elastischen Bereiches gedehnt werden.

Aus EP 1 252 015 B1 ist ein Windelverschlusselement bekannt, das eine elastische Folie als Kernschicht und beidseitig auf die Kernschicht aufkaschierte Nonwovenschichten aufweist. Die Nonwovenschichten und die Kernschicht besitzen die gleichen Außenabmessungen, d. h. die elastische Kernschicht ist in Dehnungsrichtung ebenso breit wie die beidseitig aufkaschierten Schichten aus Nonwoven. Der Anschlussbereich für das Verschlussteil ist ebenso wie der Anschlussbereich zur Befestigung an einer Windel durch eine Schicht aus einem nichtelastischen Polymer versteift. Die Versteifung erfolgt mittels einer zugfesten Folie, z. B. aus einem Polypropylen-Homopolymer, welche in den Anschlussbereichen zwischen der elastischen Folie und einer der beiden Deckschichten einkaschiert ist und die Elastizität der elastischen Kernschicht blockiert. Da elastische Polymere teure Werkstoffe sind, besteht ein Bedürfnis, den Anteil des elastischen Polymers im Verbund so gering als möglich zu halten, ohne dass sich dies nachteilig auf die Elastizität und die mechanischen Eigenschaften des Verbundmaterials auswirkt.

Aus EP 1 021 153 B1 ist ein Windelverschlusselement bekannt, das einen Träger mit elastischen und nichtelastischen Bereichen aufweist. Der Träger besteht insbesondere aus einer coextrudierten Folie mit einer elastischen Kernschicht und nichtelastischen Deckschichten. Auf einer Seite des Trägers ist ein textiles Material aufkaschiert. Auf der anderen Seite des Trägers ist ein Verschlussteil befestigt. Durch eine lokale Verstreckung, bei der bereichsweise die nichtelastischen Deckschichten des Trägers und das aufkaschierte Nonwoven überdehnt werden, wird ein elastisch dehnbarer Abschnitt erzeugt. Dies wird als selektive mechanische Aktivierung bezeichnet. Nachteilig ist auch hier, dass der elastische Träger sich über die gesamte Breite des Windelverschlusselementes einschließlich der nichtelastischen Anschlussbereiche erstreckt. Ferner ist nachteilig, dass das Windelverschlusselement nur einseitig eine textile Oberfläche aufweist.

In US 6 875 710 B2 wird ein Windelverschlusselement mit einem textilen Träger, z. B. aus Nonwoven beschrieben. In vorgegebenen Bereichen ist der Träger durch eine im thermoplastischen Zustand aufgetragene Schicht aus einem nichtelastischen Polymer verstärkt, welches die Faserstruktur der Nonwovenschicht zumindest teilweise durchsetzt. In einem anderen, davon beabstandeten Abschnitt weist der Träger eine Beschichtung aus einem thermoplastischen Elastomer auf, welche die Faserstruktur des Nonwovens ebenfalls zumindest teilweise durchsetzt und einen elastisch dehnbaren Abschnitt bildet. Wird das Material bis zur Dehngrenze des elastischen Bereiches gedehnt, besteht die Gefahr, dass das ausschließlich aus Nonwoven bestehende Material zwischen dem elastischen Bereich und den nichtelastischen Bereichen plastisch verformt und zerstört wird.

Der Erfindung liegt die Aufgabe zugrunde, ein kostengünstiges Verfahren zur Herstellung von elastischen Windelverschlusselementen anzugeben, die einen biegesteifen und zugfesten Anschlussbereich für ein Verschlussteil aufweisen.

Gegenstand dieser Erfindung und Lösung dieser Aufgabe ist ein Verfahren nach Anspruch 1.

Bei dem erfindungsgemäßen Verfahren werden Streifen aus einer Verstärkungsfolie, die eine Trägerschicht und an mindestens einer Seite einer Außenschicht aus Polyethylen aufweist, zwischen die Deckschichten eingebracht. Die aus der Verstärkungsfolie bestehenden Streifen überbrücken jeweils den Abstand zwischen zwei elastischen Folienstreifen und werden in beidseitigen Überlappungsbereichen mit den angrenzenden elastischen Folienstreifen verbunden. Die aus Nonwoven bestehenden Deckschichten werden ferner mit der Verstärkungsfolie durch Ultraschallschweißen oder thermisches Schweißen verbunden. Die Deckschicht, die mit der Polyethylenschicht der Verstärkungsschicht verbunden wird, besteht aus Bikomponentenfasern, welche einen Faserkern aus Polypropylen und einen Fasermantel aus Polyethylen aufweisen. Die Trägerschicht der Verstärkungsfolie besteht aus einem Polymer, welches sich im Vergleich zu den mechanischen Eigenschaften der elastischen Folienstreifen durch eine größere Biegesteifigkeit und Zugfestigkeit auszeichnet und einen höheren Schmelzpunkt aufweist als das Polyethylen der Außenschicht. Vorzugsweise besteht die Trägerschicht aus einem Polypropylen, insbesondere Polypropylen-Homopolymer. Zur Verwendung als Trägerschicht sind ferner Cycloolefin-Copolymere, Styrol-Polymere, Polyamide, Polylactide, thermoplastische Polyurethane sowie Mischungen der genannten Polymere geeignet.

Die aus Polyethylen bestehende Außenschicht der Verstärkungsfolie verbindet sich an den thermisch oder durch Ultraschall erzeugten Schweißstellen fest mit den ebenfalls aus Polyethylen bestehendem Fasermantel der Bikomponentenfasern, aus den die Nonwoven-Deckschicht gefertigt ist. Durch lokales Aufschmelzen gehen die Materialen eine feste Verbindung ein, wobei ein gleichmäßiges Muster von punktförmigen, ellipsenförmigen oder stabförmigen Verbindungsstellen ausgebildet wird. Aufgrund des niedrigen Schmelzpunktes von Polyethylen ist ein geringer Energieeintrag im Ultraschallschweißen oder thermischen Schweißen ausreichend. Das Ultraschallschweißen wird so betrieben, dass die Trägerschicht der Verstärkungsfolie sowie der aus Polypropylen bestehende Kern der Bikomponentenfasern nicht aufgeschmolzen wird. Entsprechendes gilt bei Anwendung eines thermischen Schweißverfahrens. Der Faserkern aus Polypropylen gewährleistet, dass die Fasern ihre Struktur behalten und nicht zerstört werden. Sowohl für die aus Polyethylen bestehende Außenschicht der Verstärkungsfolie als auch für das Mantelmaterial der Bikomponentenfasern der Nonwoven-Deckschicht können insbesondere Polyethylen in niedriger Dichte (LD-PE), lineare Polyethylene niedriger Dichte (LLD-PE) oder Polyethylene sehr niedriger Dichte (VLD-PE) eingesetzt werden.

Die Polyethylene weisen eine Schmelzetemperatur im Bereich zwischen 105° C und 145° C auf, während die Schmelzetemperatur von Polypropylen höher ist.

Die Trägerschicht der Verstärkungsfolie verleiht dem Material in den Materialabschnitten, die einen biegesteifen und zugfesten Anschlußbereich für Verschlussteile bilden, die gewünschten mechanischen Eigenschaften und ist insbesondere maßgebend für die Weiterreißfestigkeit und Biegesteifigkeit des betreffenden Bereiches. Es versteht sich, dass die Trägerschicht der Verstärkungsfolie eine ausreichende Schichtstärke aufweisen muss, um die gewünschte Versteifung zu erreichen, während die Außenschicht der Verstärkungsfolie nur eine zuverlässige thermische Verbindung mit der aus Nonwoven bestehenden Deckschicht sicherstellen muss. Die aus Polyethylen bestehende Außenschicht der Verstärkungsfolie weist zweckmäßig eine Schichtstärke zwischen 2 und 20 µm auf, während die zur Versteifung vorgesehene Trägerschicht eine Dicke zwischen 20 und 100 µm haben sollte.

Die aus Bikomponentenfasern des Kern-Mantel-Typs bestehende Deckschicht weist günstigere Gebrauchseigenschaften auf als ein Nonwoven aus Polypropylenfasern. Die Polyethylenanteile der Bikomponentenfasern verbessern die Zähigkeit und Bruchdehnung des Nonwovens und geben dem Nonwoven ein besseres Reib- und Verschleißverhalten. Das aus Bikomponentenfasern gebildete Nonwoven, dessen Fasern einen Faserkern aus Polypropylen und einen Fasermantel aus Polyethylen aufweisen, behält seine Faserstruktur, wenn die Materialbahn zur Verbesserung der Elastizität mechanisch aktiviert wird. Bei einer mechanischen Aktivierung wird die Materialbahn durch eine Profilwalzenanordnung geführt, die auf die elastisch dehnbaren Bereiche der Materialbahn einwirkt und diese lokal so stark überstreckt, dass Polypropylenfasern reißen. Durch den zäheren Polyethylenmantel der Bikomponentenfasern kann die Faserstruktur trotz der Überdehnung bei einer mechanischen Aktivierung aufrechterhalten werden.

Die Verstärkungsfolie kann aus einer Coextrusionsfolie bestehen, deren Trägerschicht aus Polypropylen besteht und mit einem aus Polypropylenfasern bestehenden Nonwoven verbunden wird. Bei dieser Ausführung des erfindungsgemäßen Verfahrens wird eine Materialbahn erzeugt, die zwei unterschiedlich ausgebildete Deckschichten aufweist. Eine erste Deckschicht besteht aus einem Nonwoven aus Bikomponentenfasern, die einen Faserkern aus Polypropylen und einen Fasermantel aus Polyethylen aufweist. Eine zweite Deckschicht, die unmittelbar mit der Trägerschicht verbunden wird, besteht aus einem Nonwoven aus Polypropylenfasern. Die Haftfestigkeit der ersten Deckschicht an der Polyethylenschicht der Verstärkungsfolie ist höher als die Haftfestigkeit der Verbindung zwischen der aus Polypropylen bestehenden Nonwovenschicht und der ebenfalls aus Polypropylen bestehenden Trägerschicht der Verstärkungsfolie. Eine gezielte Reduzierung der Haftfestigkeit an einerseits der Verstärkungsfolie kann sich auf die Weiterreißfestigkeit des Laminats positiv auswirken.

Die Verstärkungsfolie kann eine oder mehrere Schichten zwischen der aus Polypropylen bestehenden Trägerschicht und der äußeren Polyethylenschicht aufweisen. Insbesondere kann die Verstärkungsfolie eine äußere Polyethylenschicht, eine Trägerschicht aus Polypropylen und eine Zwischenschicht zur Versteifung aus einem steifen Polymer aufweisen.

Die Verstärkungsfolie kann auch aus einer mehrschichtigen Folie bestehen, welche die Trägerschicht als Kern und auf beiden Seiten eine Auflage aus Polyethylen aufweist. Für beide Deckschichten werden Nonwoven aus Bikomponentenfasern verwendet, die einen Faserkern aus Polypropylen und einen Fasermantel aus Polyethylen aufweisen. Der Energieeintrag der Ultraschallschweißung wird so abgestimmt, dass die aus Polyethylen bestehenden Kontaktflächen zumindest anschmelzen oder aufschmelzen, wobei eine innige Verbindung zwischen den Mantel der Bikomponentenfasern und der angrenzenden Polyethylenauflage der Verstärkungsfolie erzeugt wird. Das Ergebnis ist eine besonders feste Verankerung der Verstärkungsstreifen in den beiden aus Nonwoven bestehenden Deckschichten.

Die elastischen Folienstreifen werden vorzugsweise mit den aus Nonwoven bestehenden Deckschichten verklebt. Das Ultraschallschweißen oder thermische Schweißen wird auf diejenigen Abschnitte des Laminats beschränkt, die bei der Handhabung eines Windelverschlusses hohen Peelkräften ausgesetzt sind.

Eine weitere vorteilhafte Ausgestaltung des erfindungsgemäßen Verfahrens sieht vor, dass die Verstärkungsfolie in den beidseitigen Überlappungsbereichen mit den elastischen Folienstreifen verklebt wird. Die Klebeverbindung in den Überlappungsbereichen begünstigt nachträglich das Ultraschallschweißen. Beim Ultraschallschweißen werden die aus Nonwoven bestehenden Deckschichten und die Verstärkungsfolie zwischen einer Sonotrode und einer Pixelwalze, deren Walzenoberfläche eine Struktur aus Erhebungen und/oder Vertiefungen aufweist, hindurchgeführt. Am Einlaufspalt kann sich Fasermaterial aufstauen. Eine randseitige Verklebung der miteinander zu verbindenden Materiallagen begünstigt die Zuführung des Materials in den Einlaufbereich zwischen Sonotrode und Pixelwalze. Um eine etwaige Faltenbildung zu verhindern, kann es vorteilhaft sein, wenn die Verstärkungsfolie mit streifenförmig aufgetragenem Klebstoff an mindestens einer Deckschicht befestigt wird, bevor die Verstärkungsfolie durch Ultraschallschweißen mit beiden Deckschichten fest verbunden wird.

Zum Ultraschallschweißen werden Sonotroden verwendet, die durch das Einleiten von hochfrequenten mechanischen Schwingungen in Resonanzschwingungen versetzt werden. Die Sonotrode wirkt auf eine Pixelwalze, deren Oberfläche eine Struktur aus Erhebungen und Vertiefungen aufweist. Die Oberflächenstruktur der Pixelwalze bestimmt die Form und Anordnung der Schweißpunkte. Das erfindungsgemäße Verfahren kann mit einer Sonotrode durchgeführt werden, die eine glatte Andruckfläche aufweist. Sofern der Energieeintrag in das Laminat zu groß ist, besteht die Gefahr, dass sich aufgeschmolzenes Polyethylen an der Sonotrode ablagert. Die Ablagerungen können lange Fahnen bilden, die sich nach gewisser Zeit ablösen und die Materialbahn verschmutzen. Die Gefahr, dass sich zwischen der Sonotrode und der Pixelwalze Material aufstaut ist geringer, wenn als Sonotrode eine walzenförmige Rotationssonotrode verwendet wird. Die Verwendung einer Rotationssonotrode stellt daher eine bevorzugte Ausführung des erfindungsgemäßen Verfahrens dar.

Gegenstand ist auch eine Materialbahn gemäß Patentanspruch 11, die durch das beschriebene Verfahren herstellbar ist.

Die Erfindung wird im Folgenden anhand einer lediglich ein Ausführungsbeispiel darstellenden Zeichnung erläutert. Es zeigen schematisch:
- **Fig. 1**: einen Längsschnitt in Dehnungsrichtung durch ein elastisches Windelverschlusselement, welches einen nicht elastischen und verstärkten Anschlussbereich zur Befestigung eine Verschlussteils aufweist,
- **Fig. 2**: eine Draufsicht auf eine Materialbahn, aus der Windelverschlusselemente gemäß Fig. 1 ausgestanzt werden können,
- **Fig. 3**: den Schichtenaufbau der Materialbahn in einem nicht elastischen und verstärkten Anschlussbereich,
- **Fig. 4**: eine Werkzeuganordnung zum Ultraschallverschweißen der in Fig. 3 dargestellten Materiallagen,
- **Fig. 5**: eine alternative Werkzeuganordnung zum Ultraschallverschweißen der in Fig. 3 dargestellten Materiallagen,
- **Fig. 6**: eine Ausführungsvariante des in Fig. 3 dargestellten Schichtenaufbaus.

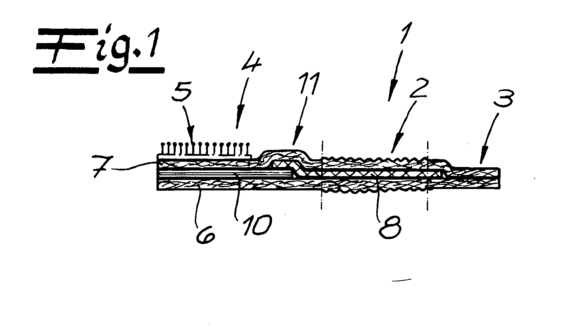

Das in Fig. 1 dargestellte Windelverschlusselement 1 weist einen elastisch dehnbaren Abschnitt 2, einen nicht elastischen Anschlussbereich 3 zur Befestigung an einer Windel sowie einen ebenfalls nicht elastischen und verstärkten Anschlussbereich 4 zum Anschluss eines Verschlussteils 5 auf. Das Windelverschlusselement ist mehrschichtig aufgebaut und weist Deckschichten 6, 7 aus Nonwoven sowie einen zwischen den Deckschichten 6, 7 einkaschierten Folienstreifen 8 aus einem elastisch dehnbaren Polymer auf. Die Deckschichten 6, 7 sind in Dehnungsrichtung breiter als der einkaschierte elastische Folienstreifen 8 und sind an überstehenden Abschnitten, welche die nicht elastische Anschlussbereiche 3, 4 bilden, miteinander verbunden. Ein erster Anschlussbereich 3 zur Befestigung an einer Windel ist weich und flexibel und setzt sich lediglich aus den beiden miteinander verbunden Nonwovenschichten 6, 7 zusammen. Der Anschlussbereich 4 für das Verschlussteil 5 hingegen wird durch eine Verstärkungsfolie 10 versteift, die in einem Überlappungsbereich 11 mit dem einkaschierten elastischen Foliestreifen 8 verbunden ist. Der Überlappungsbereich 11 ist etwa 2 mm bis 10 mm breit. Die Verstärkungsfolie 10 besteht aus einem Polymer, welche sich im Vergleich zu den mechanischen Eigenschaften des elastischen Folienstreifens 8 durch eine wesentlich größere Biegesteifigkeit und größere Zugfestigkeit auszeichnet. Der elastisch dehnbare Abschnitt 2 des Windelverschlusselementes ist durch eine monoaxiale Verstreckung des Laminats aktiviert worden. Durch eine lokal begrenzte Verstreckung werden die Fasern der Nonwovenschichten 6, 7 durch die mechanische Aktivierung überdehnt, wobei der Dehnungswiderstand des Laminats in diesem Bereich reduziert wird. Der verstreckte Bereich ist schmaler als der elastische Folienstreifen und endet vor dem Überlappungsbereich 11.

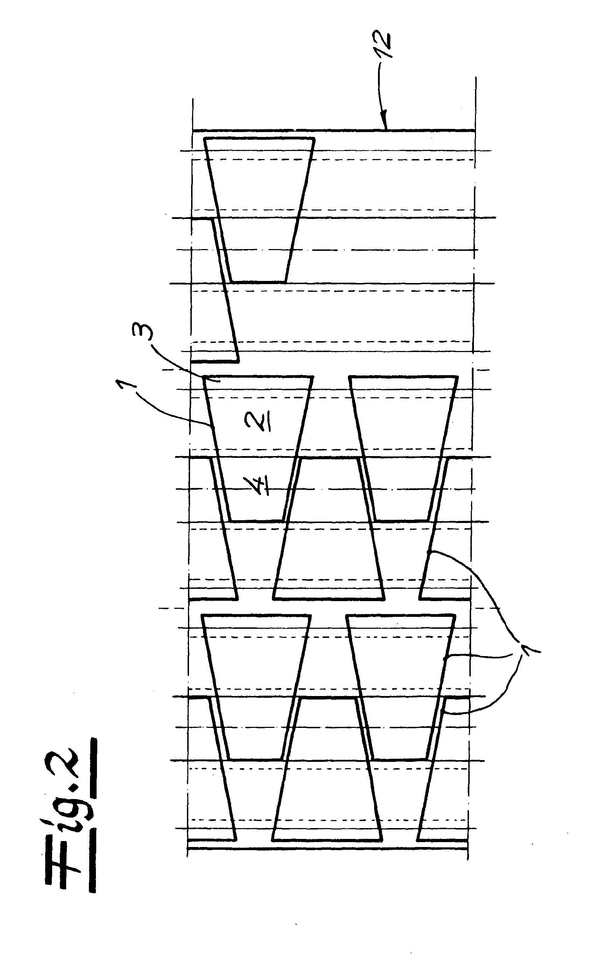

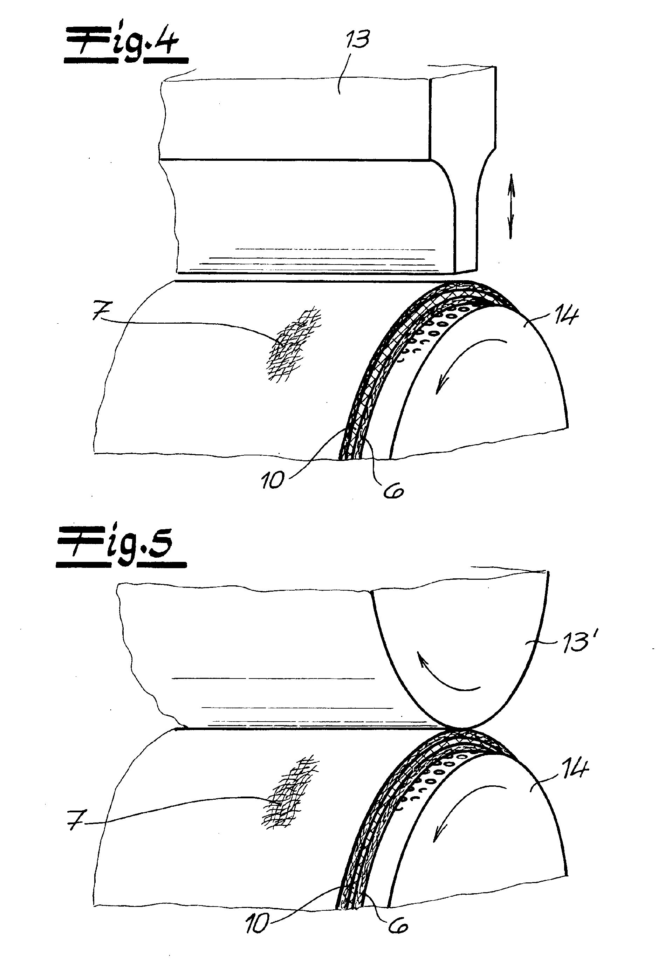

Die Windelverschlusselemente 1 werden aus einer breiten Materialbahn 12 ausgestanzt, die in Fig. 2 in einer Draufsicht dargestellt ist. Zur Herstellung der in Fig. 2 dargestellten Materialbahn 12 werden parallel und zueinander beabstandete Folienstreifen 8 aus einem elastisch dehnbaren Polymer zwischen zwei Deckschichten 6, 7 aus Nonwoven einkaschiert. Ferner werden Streifen aus einer Verstärkungsfolie 10 zwischen die Deckschichten 6, 7 eingebracht, die jeweils den Abstand zwischen zwei elastischen Folienstreifen 8 überbrücken und in beidseitigen Überlappungsbereichen 11 mit den angrenzenden elastischen Folienstreifen 8 verklebt werden. Die aus Nonwoven bestehenden Deckschichten 6, 7 werden mit der Verstärkungsfolie durch Ultraschallschweißen verbunden. Dazu werden die aus Nonwoven bestehenden Deckschichten 6, 7 und die Verstärkungsfolie 10 zwischen einer Sonotrode 13, 13' und einer Pixelwalze 14, deren Walzenoberfläche eine Struktur oder Erhebung und/oder Vertiefungen aufweist, hindurchgeführt. In den Fig. 4 und 5 sind die Werkzeuganordnungen für das Ultraschallschweißen dargestellt. Im Ausführungsbeispiel der Fig. 4 wird eine stehende Sonotrode 13 mit einer glatten Andruckfläche verwendet. Im Ausführungsbeispiel der Fig. 5 wird als Sonotrode eine walzenförmige Rotationssonotrode 13' verwendet.

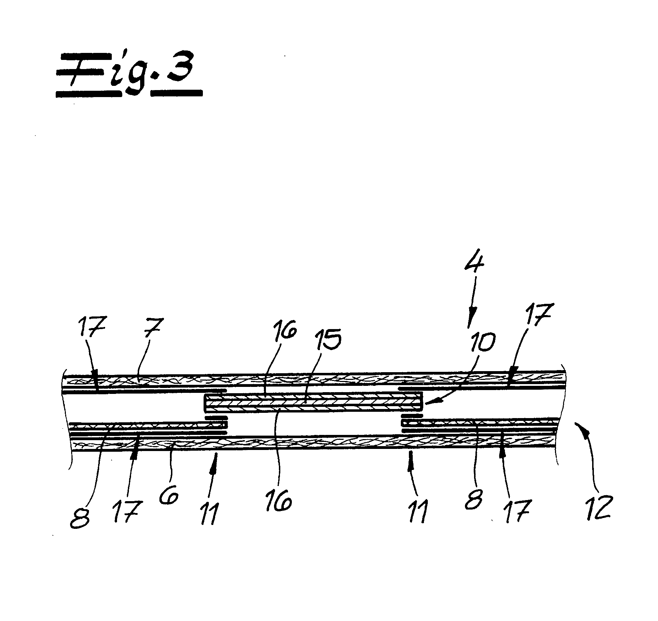

Der Schichtenaufbau der Materialbahn 12 in dem nicht elastischen und verstärkten Anschlussbereich 4 ist in Fig. 3 vergrößert dargestellt. Die Verstärkungsfolie 10 weist eine Trägerschicht 15 aus Polypropylen und auf beiden Seiten eine Außenschicht 16 aus Polyethylen auf. Die beiden Nonwoven-Deckschichten 6, 7 bestehen aus Bikomponentenfasern, die einen Kern aus Polypropylen und einen Mantel aus Polyethylen aufweisen. Die Nonwovenschichten 6, 7 sind mit den aus Polyethylen bestehenden Außenschichten 16 der Verstärkungsfolien 10 durch Ultraschallschweißen verbunden worden. In den beidseitigen Überlappungsbereichen 11 ist die Verstärkungsfolie 10 mit den elastischen Folienstreifen 8 verklebt. Die elastischen Folienstreifen 8 sind ihrerseits mit den Deckschichten 6, 7 an Klebeflächen 17 verklebt.

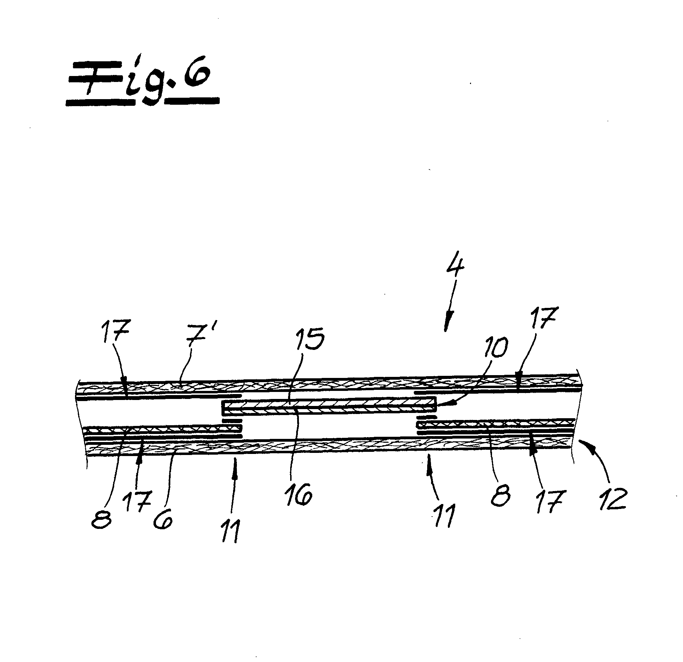

Im Ausführungsbeispiel der Fig. 6 weist die Verstärkungsfolie 10 eine Trägerschicht 15 aus Polypropylen und auf nur einer Seite eine Außenschicht 16 aus Polyethylen auf. Die Deckschicht 6, die mit der Polyethylenschicht der Verstärkungsfolie 10 verbunden ist, besteht wiederum aus Bikomponentenfasern, welche einen Faserkern aus Polypropylen und einen Fasermantel aus Polyethylen aufweisen. Die Trägerschicht 15 der Verstärkungsfolie 10 ist mit einem aus Polypropylenfasern bestehenden Nonwoven 7' verbunden.

## Patentansprüche

1. Verfahren zur Herstellung einer Materialbahn (12), aus der elastisch dehnbare Windelverschlusselemente (1) mit einem nicht elastischen und verstärkten Anschlussbereich (4) zur Befestigung eines Verschlussteils (5) ausstanzbar sind, wobei parallele und zueinander beabstandete Folienstreifen (8) aus einem elastisch dehnbaren Polymer zwischen zwei Deckschichten (6, 7) aus Nonwoven einkaschiert werden, **dadurch gekennzeichnet, dass** Streifen aus einer Verstärkungsfolie (10), die eine polymere Trägerschicht (15) und an mindestens einer Seite eine Außenschicht (16) aus Polyethylen aufweist, zwischen die Deckschichten (6, 7) eingebracht werden, wobei die aus der Verstärkungsfolie (10) bestehenden Streifen jeweils den Abstand zwischen zwei elastischen Folienstreifen (8) überbrücken und in beidseitigen Überlappungsbereichen (11) mit den angrenzenden Folienstreifen (8) verbunden werden, und dass die aus Nonwoven bestehenden Deckschichten (6, 7) mit der Verstärkungsfolie (10) durch Ultraschallschweißen oder thermisches Schweißen verbunden werden, wobei die Deckschicht (6), die mit der aus Polyethylen bestehenden Außenschicht (16) der Verstärkungsfolie (10) verbunden wird, aus Bikomponentenfasern besteht, welche einen Faserkern aus Polypropylen und einen Fasermantel aus Polyethylen aufweisen, und wobei die Trägerschicht (15) der Verstärkungsfolie (16) aus einem Polymer besteht, welches einen höheren Schmelzpunkt aufweist als das Polyethylen der Außenschicht (16).

2. Verfahren nach Anspruch 1, **dadurch gekennzeichnet, dass** die Trägerschicht (15) aus einem Polypropylen, insbesondere Polypropylen-Homoplymer, Cycloolefin-Copolymer, Styrol-Polymer, Polyamid, Polylactid, thermoplastischem Polyurethan oder Mischungen der vorgenannten Polymere besteht.

3. Verfahren nach Anspruch 1, **dadurch gekennzeichnet, dass** die Trägerschicht (15) der Verstärkungsfolien (10) aus einem Polypropylen besteht und mit einem aus Polypropylenfasern bestehenden Nonwoven (7') verbunden wird.

4. Verfahren nach Anspruch 1 oder 2, **dadurch gekennzeichnet, dass** die Verstärkungsfolie (10) aus einer mehrschichtigen Folie besteht, welche die Trägerschicht (15) als Kern und auf beiden Seiten eine Außenschicht (16) aus Polyethylen aufweist, und dass für die beiden Deckschichten (6, 7) Nonwoven aus Bikomponentenfasern, die einen Faserkern aus Polypropylen und einen Fasermantel aus Polyethylen aufweisen, verwendet werden.

5. Verfahren nach einem der Ansprüche 1 bis 4, **dadurch gekennzeichnet, dass** die elastischen Folienstreifen (8) mit den Deckschichten (6, 7) verklebt werden.

6. Verfahren nach einem der Ansprüche 1 bis 5, **dadurch gekennzeichnet, dass** die Verstärkungsfolie (10) in den beidseitigen Überlappungsbereichen (11) mit den elastischen Folienstreifen (8) verklebt wird.

7. Verfahren nach einem der Ansprüche 1 bis 6, **dadurch gekennzeichnet, dass** die Verstärkungsfolie (10) mit streifenförmig aufgetragenem Klebstoff an mindestens einer Deckschicht befestigt wird, wobei die Verstärkungsfolie (10) durch Ultraschallschweißen oder thermisches Schweißen (6, 7) mit beiden Deckschichten fest verbunden wird.

8. Verfahren nach einem der Ansprüche 1 bis 7, **dadurch gekennzeichnet, dass** die aus Nonwoven bestehenden Deckschichten (6, 7) und die Verstärkungsfolie (10) zum Ultraschallschweißen zwischen einer Sonotrode (13, 13') und einer Pixelwalze (14), deren Walzenoberfläche eine Struktur aus Erhebungen und/oder Vertiefungen aufweist, hindurchgeführt werden.

9. Verfahren nach Anspruch 8, **dadurch gekennzeichnet, dass** eine stehende Sonotrode (13) mit einer glatten Andruckfläche verwendet wird.

10. Verfahren nach Anspruch 8, **dadurch gekennzeichnet, dass** als Sonotrode eine walzenförmige Rotationssonotrode (13') verwendet wird.

11. Materialbahn (12), aus der elastisch dehnbare Windelverschlusselemente (1) mit einem nicht elastischen und verstärkten Anschlussbereich (4) zur Befestigung eines Verschlussteils (5) ausstanzbar sind, mit parallele und zueinander beabstandete Folienstreifen (8) aus einem elastisch dehnbaren Polymer, die zwischen zwei Deckschichten (6, 7) aus Nonwoven einkaschiert sind, **dadurch gekennzeichnet, dass** Streifen aus einer Verstärkungsfolie (10), die eine polymere Trägerschicht (15) und an mindestens einer Seite eine Außenschicht (16) aus Polyethylen aufweist, zwischen den Deckschichten (6, 7) angeordnet sind, wobei die aus der Verstärkungsfolie (10) bestehenden Streifen jeweils den Abstand zwischen zwei elastischen Folienstreifen (8) überbrücken und in beidseitigen Überlappungsbereichen (11) mit den angrenzenden Folienstreifen (8) verbunden sind, und dass die aus Nonwoven bestehenden Deckschichten (6, 7) mit der Verstärkungsfolie (10) durch Ultraschallschweißen oder thermisches Schweißen verbunden sind, wobei die Deckschicht (6), die mit der aus Polyethylen bestehenden Außenschicht (16) der Verstärkungsfolie (10) verbunden ist, aus Bikomponentenfasern besteht, welche einen Faserkern aus Polypropylen und einen Fasermantel aus Polyethylen aufweisen, und wobei die Trägerschicht (15) der Verstärkungsfolie (16) aus einem Polymer besteht, welches einen höheren Schmelzpunkt aufweist als das Polyethylen der Außenschicht (16).

## Claims

1. A method for manufacturing a web material (12) out of which elastically extensible nappy closure elements (1) having a non-elastic and reinforced attachment region (4) for fixing a closure part (5) can be stamped, wherein parallel and mutually separated foil strips (8) formed from an elastically extensible polymer are laminated between two cover layers (6, 7) formed from a nonwoven, **characterized in that** strips formed from a reinforcing foil (10) which comprises a polymer support layer (15) and an outer layer (16) formed from polyethylene on at least one side are introduced between the cover layers (6, 7), wherein the strips consisting of the reinforcing film (10) each span the distance between two elastic foil strips (8) and are bonded to the adjacent elastic foil strips (8) at overlapping regions (11) on both sides, and **in that** the cover layers (6, 7) consisting of nonwovens are bonded to the reinforcing film (10) by ultrasonic welding or thermal welding, wherein the cover layer (6), which is bonded to the outer layer (16) which consists of polyethylene, of the reinforcing layer (10), consists of bicomponent fibres which comprise a fibre core formed from polypropylene and a fibre sheath formed from polyethylene, and wherein the support layer (15) of the reinforcing film (16) consists of a polymer which has a higher melting point than the polyethylene of the outer layer (16).

2. The method as claimed in claim 1, **characterized in that** the support layer (15) consists of a polypropylene, in particular a polypropylene homopolymer, cycloolefin copolymer, styrene polymer, polyamide, polylactide, thermoplastic polyurethane or mixtures of said polymers.

3. The method as claimed in claim 1, **characterized in that** the support layer (15) of the reinforcing film (10) consists of a polypropylene and is bonded to a nonwoven (7') consisting of polypropylene fibres.

4. The method as claimed in claim 1 or claim 2, **characterized in that** the reinforcing film (10) consists of a multi-layered film which comprises the support layer (15) as the core and an outer layer (16) formed from polyethylene on both sides, and **in that** nonwovens formed from bicomponent fibres which comprise a fibre core formed from polypropylene and a fibre sheath formed from polyethylene are used for both cover layers (6, 7).

5. The method as claimed in one of claims 1 to 4, **characterized in that** the elastic foil strips (8) are bonded to the cover layers (6, 7).

6. The method as claimed in one of claims 1 to 5, **characterized in that** the reinforcing film (10) is bonded to the elastic foil strips (8) in the overlapping regions (11) on both sides.

7. The method as claimed in one of claims 1 to 6, **characterized in that** the reinforcing film (10) with adhesive applied thereto in lines is fixed to at least one cover layer, wherein the reinforcing film (10) is firmly bonded to both cover layers (6, 7) by ultrasonic welding or thermal welding.

8. The method as claimed in one of claims 1 to 7, **characterized in that** for the purposes of ultrasonic welding, the cover layers (6, 7) consisting of a nonwoven and the reinforcing film (10) are fed between a sonotrode (13, 13') and a pixel roller (14) the roller surface of which comprises a structure of elevations and/or depressions.

9. The method as claimed in claim 8, **characterized in that** a stationary sonotrode (13) with a smooth contact surface is used.

10. The method as claimed in claim 8, **characterized in that** a roller-shaped rotary sonotrode (13') is used as the sonotrode.

11. A web material (12) out of which elastically extensible nappy closure elements (1) having a non-elastic and reinforced attachment region (4) for fixing a closure part (5) can be stamped, with parallel and mutually separated foil strips (8) formed from an elastically extensible polymer laminated between two cover layers (6, 7) formed from a nonwoven, **characterized in that** strips formed from a reinforcing foil (10) which comprises a polymer support layer (15) and an outer layer (16) formed from polyethylene on at least one side are disposed between the cover layers (6, 7), wherein the strips consisting of the reinforcing film (10) each span the distance between two elastic foil strips (8) and are bonded to the adjacent elastic foil strips (8) at overlapping regions (11) on both sides, and **in that** the cover layers (6, 7) consisting of nonwovens are bonded to the reinforcing film (10) by ultrasonic welding or thermal welding, wherein the cover layer (6), which is bonded to the outer layer (16) which consists of polyethylene, of the reinforcing layer (10), consists of bicomponent fibres which comprise a fibre core formed from polypropylene and a fibre sheath formed from polyethylene, and wherein the support layer (15) of the reinforcing film (16) consists of a polymer which has a higher melting point than the polyethylene of the outer layer (16).

## Revendications

1. Procédé de fabrication d'un lé de matière (12), dans lequel on peut découper des éléments de fermeture (1) de couche élastiquement extensibles avec une zone de raccord (4) renforcée et non élastique pour la fixation d'une pièce de fermeture (5) lors duquel on contrecolle des rubans de film (8) parallèles et écartés les uns des autres en un polymère élastiquement extensible entre deux couches de recouvrement (6, 7) en non-tissé, **caractérisé en ce qu'**on introduit entre les couches de recouvrement (6, 7) des rubans en un film de renfort (10) qui comporte une couche porteuse (15) en polymère et sur au moins une face une couche extérieure (16) en polyéthylène, les rubans constitués du film de renfort (10) chevauchant chacun l'écart entre deux rubans de film (8) élastiques et dans des zones de chevauchement (11) bilatérales, étant reliés avec les rubans de film (8) adjacents et les couches de recouvrement (6, 7) constituées de non-tissé étant reliées avec le film de renfort (10) par soudage aux ultrasons ou par soudage thermique, la couche de recouvrement (6), que l'on relie avec la couche extérieure (16) constituée de polyéthylène du film de renfort étant constituée de fibres bicomposants qui comportent un coeur de fibre en polypropylène et un enrobage de fibre en polyéthylène et la couche porteuse (15) du film de renfort (16) étant constituée d'un polymère qui a un point de fusion plus élevé que le polyéthylène de la couche extérieure (16).

2. Procédé selon la revendication 1, **caractérisé en ce que** la couche porteuse (15) est constituée d'un polypropylène, notamment d'un homopolymère de polypropylène, d'un copolymère de cyclo-oléfine, d'un polymère de styrène, de polyamide, de polyactide, de polyuréthane thermoplastique, ou de mélanges des polymères précédemment cités.

3. Procédé selon la revendication 1, **caractérisé en ce que** la couche porteuse (15) des films de renfort (10) est constituée d'un polypropylène et **en ce qu'**on la relie avec un non-tissé (T) constitué de fibres de polypropylène.

4. Procédé selon la revendication 1 ou la revendication 2, **caractérisé en ce que** le film de renfort (10) est constitué d'un film multicouches qui comporte la couche porteuse (15) en tant que coeur et sur les deux faces une couche extérieure (16) en polyéthylène et **en ce que** pour les deux couches de recouvrement (6, 7), on utilise du non-tissé en fibres bicomposants qui comportent un coeur de fibre en polypropylène et un enrobage de fibre en polyéthylène.

5. Procédé selon l'une quelconque des revendications 1 à 4, **caractérisé en ce qu'**on colle les rubans de film (8) élastique avec les couches de recouvrement (6, 7).

6. Procédé selon l'une quelconque des revendications 1 à 5, **caractérisé en ce qu'**on colle le film de renfort (10) dans les zones de chevauchement (11) bilatérales avec les rubans de film (8) élastique.

7. Procédé selon l'une quelconque des revendications 1 à 6, **caractérisé en ce que** qu'avec une colle appliquée en forme de bande, on fixe le film de renfort (10) sur au moins une couche de recouvrement, alors qu'on relie fixement le film de renfort (10) par soudage aux ultrasons ou par soudage thermique (6, 7) avec les deux couches de recouvrement.

8. Procédé selon l'une quelconque des revendications 1 à 7, **caractérisé en ce que** pour le soudage aux ultrasons, on fait passer les couches de recouvrement (6, 7) constituées de non-tissé et le film de renfort (10) entre une sonotrode (13, 13') et un cylindre à pixels (14), dont la surface de cylindre présente une structure en élévations et/ou en creux.

9. Procédé selon la revendication 8, **caractérisé en ce qu'**on utilise une sonotrode (13) avec une surface de pressage lisse.

10. Procédé selon la revendication 8, **caractérisé en ce qu'**on utilise en tant que sonotrode une sonotrode de rotation (13") en forme de cylindre.

11. Lé de matière (12), dans lequel on peut découper des éléments de fermeture (1) de couche élastiquement extensibles avec une zone de raccord (4) renforcée et non élastique pour la fixation d'une pièce de fermeture (5), avec des rubans de film (8) parallèles et écartés les uns des autres en un polymère élastiquement extensible qui sont contrecollés entre deux couches de recouvrement (6, 7) en non-tissé, **caractérisé en ce que** des rubans en un film de renfort (10), qui comporte une couche porteuse (15) en polymère et sur au moins une face une couche extérieure (16) en polyéthylène sont disposés entre les couches de recouvrement (6, 7), les rubans constitués du film de renfort (10) chevauchant chacun l'écart entre deux rubans de film (8) élastiques et dans des zones de chevauchement (11) bilatérales, étant reliés avec les rubans de film (8) adjacents et les couches de recouvrement (6, 7) constituées de non-tissé étant reliées avec le film de renfort (10) par soudage aux ultrasons ou par soudage thermique, la couche de recouvrement (6), qui est reliée avec la couche extérieure (16) constituée de polyéthylène du film de renfort étant constituée de fibres bicomposants qui comportent un coeur de fibre en polypropylène et un enrobage de fibre en polyéthylène et la couche porteuse (15) du film de renfort (16) étant constituée d'un polymère qui a un point de fusion plus élevé que le polyéthylène de la couche extérieure (16).
